(19) Europäisches Patentamt
European Patent Office
Office européen des brevets

(11) Veröffentlichungsnummer: **0 129 822**
**A2**

(12) # EUROPÄISCHE PATENTANMELDUNG

(21) Anmeldenummer: 84106976.8

(22) Anmeldetag: 18.06.84

(51) Int. Cl.⁴: A 61 K 31/35

(30) Priorität: 22.06.83 DE 3322337

(43) Veröffentlichungstag der Anmeldung: 02.01.85 Patentblatt 85/1

(84) Benannte Vertragsstaaten: AT CH DE FR GB LI NL SE

(71) Anmelder: Kali-Chemie Pharma GmbH
Hans-Böckler-Allee 20 Postfach 220
D-3000 Hannover 1(DE)

(72) Erfinder: Thies, Peter Willibrord, Dr. Phil.
Ihmeplatz 6
D-3000 Hannover 91(DE)

(74) Vertreter: Lauer, Dieter, Dr.
c/o Kali-Chemie AG Postfach 220 Hans-Böckler-Allee 20
D-3000 Hannover 1(DE)

(54) **Neue pharmazeutische Zubereitungen mit Antitumorwirkung.**

(57) Es werden die Verwendung von Valepotriaten in tumorhemmend wirksamen Tagesdosen von mindestens 700 mg pro Tag zur prophylaktischen und kurativen Behandlung von Tumoren, insbesondere malignen Tumoren wie Karzinomen sowie Valepotriate in hoch dosierter Form enthaltene Arzneimittel zur Tumorbehandlung beschrieben.

EP 0 129 822 A2

Kali-Chemie Pharma GmbH
3000 Hannover

# Neue pharmazeutische Zubereitungen mit Antitumorwirkung

Die vorliegende Erfindung betrifft die Verwendung von Valepotriaten zur Prophylaxe und Behandlung von Tumorwachstum in größeren Säugetieren und Menschen, sowie tumorhemmend wirksame Arzneimittel, welche Valepotriate als tumorhemmende Wirkstoffe enthalten.

Valepotriate im Sinne dieser Anmeldung sind Ester von Polyhydroxycyclopenta(c)pyran-Verbindungen der folgenden Formel I

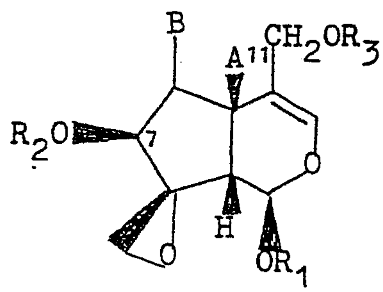

worin A und B je Wasserstoff bedeuten oder gemeinsam eine Bindung bilden und von den Resten $R_1$ bis $R_3$ einer für Isovaleryl, ein weiterer für Acetyl und der dritte für den Acylrest einer der folgenden Säuren steht: Isovaleriansäure, β-Methylvaleriansäure, α-Isovaleroxyisovaleriansäure, α-Acetoxyisovaleriansäure, β-Acetoxyisovaleriansäure, β-Acetoxy-β-methylvaleriansäure oder β-Hydroxyisovaleriansäure.

Die Valepotriate sind bekannte Verbindungen (s. z.B. Thies et al in Planta medica 41, 15-20 (1981)), welche pharmakologisch aktive Inhaltsstoffe des Baldrians bzw. von Pflanzen aus der Familie der Valerianaceen oder Abkömmlinge derselben darstellen. Die Valepotriate sind bekannt für ZNS-wirksame Eigenschaften, insbesonde beruhigende und psychisch ausgleichende und auch spasmolytische Eigenschaften.

Die beruhigenden und psychisch ausgleichenden Wirkungen des Baldrians und seiner Inhaltsstoffe werden medizinisch seit langem genutzt. In pharmazeutischen Präparaten zur Beruhigung und Äquilierung vegetativer und psychischer Störungen werden Baldrianextrakte bzw. Valepotriate üblicherweise in Mengen eingesetzt, welche Einzeldosen von 1-50 mg Valepotriat entsprechen.

Bounthan et al berichten in einer Studie (Planta medica 41, 21-28 (1981)), daß einige Valepotriate in in vitro Testen cytotoxische Eigenschaften gegenüber Hepatoma-Zellen zeigten und Didrovaltratum sich in einem in vivo Test nach i.p.-Applikation in Mäusen gegen experimentell erzeugte Krebs-II-Ascites-Tumorzellen wirksam zeigte.

Der vorliegenden Erfindung liegt die Aufgabe zugrunde, neue pharmazeutische Zubereitungen mit tumorhemmender Wirkung zur Prophylaxe und Behandlung von Tumoren, insbesondere malignen Tumoren, wie Karzinomen in Menschen und größeren Säugetieren zu entwickeln.

Es wurde nun überraschend gefunden, daß die oben definierten Valepotriate in Dosen, welche ein Vielfaches der bisher klinisch verwandten Dosen betragen, nämlich in Tagesdosen von mindestens 700 mg, in Menschen und größeren Säugetieren tumorhemmend wirksam sind.

Als tumorhemmende Wirkstoffe können die Valepotriate einzeln oder als Gemische angewendet werden, z.B. als Valepotriat-Gemische, welche aus Extrakten von Wurzeln und Rhizomen verschiedener Valerianaceen- und Kentranthusarten gewonnen werden. Die wichtigsten Vertreter der Valepotriate sind Didrovaltratum, Valtratum, Isovaltratum und Acevaltratum. Weitere Beispiele sind 7-Homovaltratum, Homoacevaltratum, 1-Homovaltratum, 11-$\alpha$-Aceisovaltratum, 1-$\alpha$-Acevaltratum, Acetoxyhydroxydidrovaltratum, Isovaleroxyhydroxydidrovaltratum, Isodidrovaltratum und Homodidrovaltratum. Als Valepotriatgemische können z.B. genuine Gemische von Valepotriaten verwendet werden, welche die Valepotriate in ihrem natürlichen Mischungsverhältnis, wie es in folgenden pharmazeutisch verwendeten Valerianaceen- und Kentranthusarten vorkommt: Valeriana officinalis L., Valeriana wallichii D.C., Valeriana jatomansii Jones, Valeriana edulis procera Mey, Kentranthus ruber D.C.

Gewünschtenfalls können auch genuine Valepotriatgemische aus Mischungen verschiedener Valerianaceen- bzw.-Kentranthusarten verwendet werden. Als günstig erweisen sich Valepotriate mit Monoen-Struktur, d.h. Verbindungen der Formel I, worin A und B je Wasserstoff bedeuten, insbesondere Didrovaltratum und Homodidrovaltratum, oder Valepotriat-Gemische, welche einen Anteil von mindestens 50 %, z.B. 50-95 % an monoenischen Verbindungen, vorwiegend Didrovaltratum, enthalten. Geeignet sind z. B. Gemische, welche 70-90 % monoenische Verbindungen, insbesondere Didrovaltratum, und/oder Homodidrovaltratum und 10-30 % dienische Verbindungen, insbesondere Valtratum und/oder Isovaltratum enthalten, beispielsweise Gemische, enthaltend 70-90 % Didrovaltratum und Homodidrovaltratum, 10-25 % Isovaltratum und/oder Valtratum und 1-7 % Acevaltratum. So ist insbesondere das genuine Valepotriatgemisch aus Valeriana wallichii D.C. geeignet.

Aufgrund ihrer spezifisch tumorhemmenden Wirkung und ihrer guten Verträglichkeit eignen sich die Valepotriate bei mehrwöchiger Anwendung von Tagesdosen von mindestens 700mg zur Prophylaxe und Behandlung von Tumoren, insbesondere malignen Tumoren, wie Karzinomen, z.B. Leberkarzinom, Kolonkarzinom, Magenkarzinom, Mammakarzinom, Lungenkarzinom, Nierenkarzinom, Uteruskarzinom oder Prostatakarzinom sowie Sarkomen und Hämoblastosen.

Die Dosierung kann je nach Art des zu behandelnden Zustandes und der verwendeten Valepotriate variieren. Gute prophylaktische und kurative Wirkungen können bei Menschen und größeren Säugetieren im allgemeinen bei mehrwöchigen Behandlungen mit vorzugsweise oral applizierten Tagesdosen von mindestens 700 mg, beispielsweise 700-2100 mg (entsprechend eine mg/kg-Dosis per os von 10-30), insbesondere Tagesdosen von 800-1500 mg erhalten werden. Diese Tagesdosen werden zweckmäßig in mehreren Teildosen, z.B. in 2-6 Teildosen, über den Tag verteilt appliziert.

In klinischen Untersuchungen an Krebspatienten zeigen die Valepotriate bei längerer Anwendung in Dosen von mindestens 700 mg täglich cancerostatische und redifferenzierende Effekte gegenüber einer Vielzahl von Krebserkrankungen. Dabei zeichnen sich die Valepotriate durch hohe Spezifität ihrer Antitumorwirkung und eine äußerst geringe Toxizität und gute Verträglichkeit aus. Keines der heute in der klinischen Anwendung befindlichen chemotherapeutischen Cancerostatika, welche bekanntlich auch allgemeine Cytostatika sind, besitzt eine derart hohe Spezifität der Antitumorwirkung. Die von den bisher klinisch gebräuchlichen chemotherapeutischen Cancerostatika bekannten starken Nebenwirkungen treten bei Behandlung mit Valepotriaten nicht auf. Als Nebenwirkungen werden lediglich eine durch

Natrium- und Chlorionen-Verlust bedingte Appetitlosigkeit, welche durch zusätzliche Kochsalzgaben völlig behoben werden kann, und eine Verminderung der intestinalen Motilität mit Neigung zur Verstopfung, welche ebenfalls problemlos symptomatisch behandelt werden kann, beobachtet.

Die Behandlung mit Valepotriaten verträgt sich gut mit bisher bei der Krebsversorgung üblichen diätetischen und/oder medikamentösen Versorgungsmaßnahmen, so daß diese während der Behandlung gewünschtenfalls beibehalten werden können.

So konnte bei klinischer Anwendung an 26 Patienten, welche an verschiedenen Krebsarten zum Teil in weit fortgeschrittenem Stadium litten, festgestellt werden, daß bei mehrwöchiger Behandlung mit oralen Tagesdosen von 800-1500 mg eines aus Valeriana wallichii D.C. gewonnenen genuinen Valepotriatgemisches, enthaltend 83,5 % eines Didrovaltratum/Homodidrovaltratum-Gemisches, 14,8 % eines Valtratum/Isovaltratumgemisches und 1,7 % Acevaltratum, eine wesentliche Verbesserung des Allgemeinzustandes (Rückgang der auf Tumoraktivität beruhenden Schmerzen, Verschiebung der tumorspezifischen pathologischen biochemischen Enzymwerte in Richtung auf Normalisierung) und ein Rückgang der Metastasen und Regression des Tumors stattfanden. Soweit die Patienten unter einer in der Krebsbehandlung üblichen diätetischen und/oder medikamentösen Schutzversorgung standen, wurde diese während der Behandlung beibehalten. Unter den behandelten Patienten befanden sich Patienten mit exzessiver Lebermetastasierung nach vorher operierten Kolonkarzinomen, Patienten mit metastasierendem Nierenkarzinom, Patientinnen mit Mammakarzinomen, Patienten mit rezidivierender Metastasierung eines früher operierten Synovialomes, einer der schwerstbehandelbaren Tumoren, Patienten mit Lungenkarzinom und Patienten mit Pigmentkarzinom. Mehrere stationäre Patienten in stark fortgeschrittenem Krankheitsstadium wurden bereits nach 4-5-wöchiger Behandlung derart gebessert, daß sie weitgehend

beschwerdefrei aus der Klinik entlassen werden konnten.

Besserungen dieser Art konnten bei den vorgenannten Krankheitszuständen weder durch eine bisher übliche Chemotherapie noch durch eine andere bisher gängige Krebstherapie ermöglicht werden.

Aufgrund der hohen Spezifität ihrer Antitumorwirkung und ihrer guten Verträglichkeit sind die Valepotriate im Gegensatz zur klassischen Chemotherapie gut für eine Langzeitanwendung geeignet. Eine Behandlung mit Valepotriaten kann in Anbetracht des Fehlens systemischer Toxizität anders als die toxische Chemotherapie schon relativ frühzeitig als Schutztherapie auf lange Zeit angewendet werden.

Die Valepotriate werden vorzugsweise oral appliziert.

Erfindungsgemäß können die Valepotriate zusammen mit üblichen pharmazeutischen Hilfs- und/oder Trägerstoffen in pharmazeutischen Zubereitungen enthalten sein. Als Beispiele oral applizierbarer Präparate seien Tabletten, insbesondere filmüberzogene Tabletten, Kapseln, Pellets, Granulate oder Dragees genannt. Der Valepotriat-Gehalt pro Einzeldosis dieser Zubereitungen liegt zweckmäßig wesentlich höher als der Valepotriat-Gehalt bisher bekannter Baldrianextrakt- und/oder valepotriathaltiger Zubereitungen. Geeignet sind z.B. Valepotriat-Gehalte von 100-800 mg, insbesondere 150-500 mg, pro Einzeldosis. Vorzugsweise sind die Valepotriate zur Stabilitätsverbesserung in mikroverkapselter Form in den Zubereitungen enthalten. Die Mikroverkapselung von Valepotriaten kann auf an sich bekannte Weise z.B. nach den in der DE-OS 28 49 029 beschriebenen Verfahren erfolgen.

Die Zubereitungen können pharmazeutisch übliche feste, anorganische und/oder organische Trägerstoffe, wie z.B.

Talkum, Milchzucker oder Stärke, oder flüssige organische Trägerstoffe, wie Öle, z.B. Triglyceridgemische von gesättigten Pflanzenfettsäuren, enthalten. Daneben können übliche pharmazeutische Hilfsstoffe enthalten sein, wie beispielsweise Gleitmittel wie Magnesiumstearat oder Tablettensprengmittel, Suspensionsmittel, ferner Konservierungsmittel, Stabilisierungsmittel, Geschmackskorrigenzien und dergleichen. Gewünschtenfalls können auch die Freisetzung der Wirkstoffe verzögernde Stoffe, wie z.B. Polyvinylacetate, Acrylat- oder Methacrylat-Copolymere, höhere Fettalkohole und andere wachsartige Substanzen enthalten sein.

Die vorzugsweise mikroverkapselten Valepotriate können mit den pharmazeutischen Hilfs- und/oder Trägerstoffen in an sich bekannter Weise gemischt und formuliert werden. Zur Herstellung fester Arzneiformen können die mikroverkapselten Wirkstoffe beispielsweise mit den Hilfs- und/oder Trägerstoffen in üblicher Weise gemischt und granuliert werden. Je nach Art der verwendeten Wirkstoffe kann gegebenenfalls auch durch einfaches Mischen ein direkt tablettierbares Pulver erhalten werden. Das Granulat oder Pulver kann direkt in Kapseln abgefüllt oder in üblicher Weise zu Tablettenkernen verpreßt werden. Diese können in bekannter Weise dragiert oder mit einem Film überzogen werden. Die Valepotriate können auch in einem flüssigen Trägerstoff suspendiert in Weichgelatinekapseln abgefüllt werden.

Zweckmäßigerweise können die Arzneiformen in an sich bekannter Weise mit einem magensaftresistenten, darmlöslichen Filmüberzug versehen werden. Als Beispiele für pharmazeutisch übliche polymere Überzugsmaterialien, welche sich erst ab pH 5,5 lösen und so die Arzneistoffe vor Magensafteinwirkung schützen und eine Reizung der Magenschleimhäute verhindern, seien anionische Methacrylsäure/Methacrylatester Polymere (z.B. Handelsprodukte Eudragit ® L und S) oder Celluloseester- und -ätherderivate, wie Celluloseacetatphthalate oder Hydroxypropylmethylcellulosephthalate genannt.

Es kann sich als günstig erweisen, die Applikation von Valepotriaten in den erfindungsgemäßen hohen Dosen mit einer zusätzlichen Applikation von Natriumchlorid zu kombinieren. Die zu verwendenden Dosen an Natriumchlorid können nach Art des zu behandelnden Zustandes, Art der applizierten Valepotriate und in Abhängigkeit der Behandlungsdauer variieren. Als geeignet haben sich Tagesdosen von 1,5-4 g Natriumchlorid erwiesen. Das Verhältnis von Chloridgaben zu Valepotriatgaben kann beispielsweise zwischen 1 : 1 und 1 : 5 liegen. Das Natriumchlorid kann par-enteral oder oral zugeführt werden, z.B. in Form gebräuchlicher Infusionslösungen appliziert oder auch oral beispielsweise in Form von Kochsalztabletten eingenommen werden.

Die Erfindung betrifft ferner ein Behältnis, welches die vorgehend beschriebenen pharmazeutischen Valepotriatzubereitungen sowie Anweisungen zur Einnahme dieser Valepotriatzubereitungen zur Prophylaxe oder Behandlung von Tumorerkrankungen enthält.

Die nachfolgenden Beispiele erläutern die Erfindung, sollen deren Umfang jedoch in keiner Weise beschränken.

## Beispiel 1: Filmüberzogene Tabletten

### A Tablettenkerne

Zusammensetzung:

| | |
|---|---|
| Mikroverkapseltes Valepotriatgemisch* | 800 Gew.-Teile |
| Lactose | 66 Gew.-Teile |
| Quervernetztes Polyvinylpyrrolidinon** | 66 Gew.-Teile |
| Magnesiumstearat | 8 Gew.-Teile |
| Gesamt | 940 Gew.-Teile |

Herstellung:

Das pulverförmige mikroverkapselte Valepotriatgemisch wird mit der Lactose vermischt und die Mischung auf einer Tablettiermaschine kompaktiert. Die erhaltenen Komprimate werden anschließend durch ein Sieb mit 2 mm Maschenweite passiert. Das erhaltene Granulat wird mit dem Polyvinylpyrrolidinon und dem Magnesiumstearat vermischt, und die Mischung wird zu Tablettenkernen von 940 mg Gewicht verpreßt. Jeder der so erhaltenen Kerne enthält 160 mg Valepotriate.

------

_* = 20 Gew.-% Valepotriate enthaltendes Pulver, auf an sich bekannte Weise nach dem in der DOS 28 49 029 beschriebenen Verfahren hergestellt durch Mikroverkapselung von 160 Gew.-Teilen eines durch Extraktion aus Valeriana wallichii D.C. gewonnenen Valepotriatgemisches, enthaltend 83,5 ± 4 Gew.-% Didrovaltratum/Homodidrovaltratum-Gemisch (enthaltend 10-15 % Homodidrovaltratum), 14,8 ± 1,5 Gew.-% Isovaltratum/Valtratum-Gemisch und 1,7 ± 0,5 Gew.-% Acevaltratum mit 215 Gew.-Teilen Gummi arabicum und 425 Gew.-Teilen Kollidon VA64 ® (Hersteller BASF Polyvinylpyrrolidon).

** = Crosporidone USP 20/NF 15 3. Supplement quervernetztes homopolymeres Vinylpyrrolidinon, (Handelsprodukt Polyplastone XL ® Hersteller GAF).

B Filmüberzogene Tabletten

Die Tablettenkerne werden entsprechend Variante B I zu magenlöslichen oder entsprechend Variante B II zu magensaftresistenten, dünndarmlöslichen Filmtabletten weiterverarbeitet.

Variante B I

Zusammensetzung:

| | | |
|---|---|---|
| Tablettenkerne | 940 | Gew.-Teile |
| Schellack | 6 | Gew.-Teile |
| Hydroxypropylmethylcellulose 2910 | 86 | Gew.-Teile |
| Polyäthylenglycol 4000 | 27 | Gew.-Teile |
| Propylenglycol | 6,5 | Gew.-Teile |
| Titandioxid | 7,5 | Gew.-Teile |
| Talkum | 13,5 | Gew.-Teile |
| Calciumcarbonat | 13,5 | Gew.-Teile |
| Gesamt | 1100 | Gew.-Teile |

Herstellung:

Die Tablettenkerne werden in einem Dragierkessel mit einer 20%-igen Lösung von Schellack in Isopropanol gleichmäßig befeuchtet und anschließend mit warmer Luft von 40 oC getrocknet. Dieser Vorgang wird solange wiederholt, bis die gesamte Menge Schellack aufgetragen worden ist.

Die Hydroxypropylmethylcellulose 2910 wird in Wasser oder einem geeigneten organischen Lösungsmittel, z. B. Isopropanol, aufgelöst. Die übrigen Hilfsstoffe werden unter

Rührung und Homogenisieren in die Mischung eingearbeitet und die entstandene Filmüberzugs-Suspension wird in einem Dragierkessel auf die mit Schellack umhüllten Kerne aufgesprüht und getrocknet.

Variante B II

Zusammensetzung:

| | | |
|---|---|---|
| Tablettenkerne | 940 | Gew.-Teile |
| Schellack | 6 | Gew.-Teile |
| Eudragit L 100 ® *** | 41,7 | Gew.-Teile |
| Dibutylphthalat | 11,5 | Gew.-Teile |
| Polyäthylenglycol 6000 | 3,5 | Gew.-Teile |
| Farblack | 2,8 | Gew.-Teile |
| Titandioxid | 8,5 | Gew.-Teile |
| Magnesiumstearat | 6,9 | Gew.-Teile |
| Talkum | 29,1 | Gew.-Teile |
| Gesamt | 1050 | Gew.-Teile |

*** = Anionisches Polymerisat aus Methacrylsäure und Methacrylatsäureestern, löslich ab pH 6, Hersteller: Röhm GmbH.

Herstellung:

Die Tablettenkerne werden, wie in Variante B I beschrieben, mit Schellack umhüllt. Eudragit L 100 wird in einem geeigneten organischen Lösungsmittel, z.B. Isopropanol, aufgelöst, die übrigen Hilfsstoffe werden unter Rühren und Homogenisieren in die Lösung eingearbeitet und die entstandene Filmüberzugs-Suspension wird in einem Dragierkessel auf die mit Schellack umhüllten Kerne aufgesprüht und getrocknet.

Beispiel 2: Kapseln

Zusammensetzung:

| | | |
|---|---|---|
| Mikroverkapseltes Valepotriatgemisch* | 518,5 | Gew.-Teile |
| Magnesiumstearat | 6,5 | Gew.-Teile |
| Gesamt | 525 | Gew.-Teile |

Herstellung:

Das pulverförmige mikroverkapselte Valepotriatgemisch wird auf einer Tablettiermaschine kompaktiert. Anschließend werden die Komprimate durch ein Sieb mit 1,6 mm Maschenweite passiert. Man vermischt das so erhaltene Granulat mit dem Magnesiumstearat und füllt die Pulvermischung in Hartgelatinekapseln der Größe 00 ein. Jede Kapsel enthält 525 mg Pulvermischung entsprechend 100 mg Valepotriaten.

Beispiel 3: Portionsbeutel

Zusammensetzung:

| | | |
|---|---|---|
| Mikroverkapseltes Valepriatgemisch* | 3200 | Gew.-Teile |
| hochdisperse Kieselsäure (Aerosil) | 16 | Gew.-Teile |
| Gesamt | 3216 | Gew.-Teile |

Herstellung:

Das pulverförmige mikroverkapselte Valepotriatgemisch wird mit dem Aerosil gemischt und die Mischung in Portionen von 3,216 g in Portionsbeutel abgefüllt. Jeder Portionsbeutel enthält 640 mg Valepotriate. Zur Herstellung von Portionsbeuteln mit einem Gehalt von 800 mg Valepotriaten werden Portionen von 4,020 g der Mischung pro Beutel abgefüllt.

Beispiel 4: Weichgelatinekapseln

A) Kapseln mit einem Valepotriatgehalt von 160 mg

Zusammensetzung:

| | |
|---|---|
| Valepotriatgemisch (= genuines Gemisch, gewonnen aus Valeriana wallichii D.C., Zusammensezung s. Fußbote zu Beispiel 1) | 160 Gew.-Teile |
| Miglyol 812 ® -**** | 148 Gew.-Teile |
| Gesamt | 308 Gew.-Teile |

**** = öliges Triglyceridgemisch von gesättigten Pflanzenfettsäuren mit C8-, C10-, und C12-Kettenlänge, Hersteller: Dynamit Nobel AG.

Herstellung:

Das Valepotriatgemisch wird unter leichtem Erwärmen und Rühren in dem Miglyol 812 aufgelöst. Die Lösung wird zu im Mittel 302 Mikroliter Lösung enthaltenden Weichgelatinekapseln verarbeitet. Valepotriatwirkstoffgehalt pro Kapsel 160 mg.

B) Kapseln mit einem Valepotriatgehalt von 240 mg

Zusammensetzung:

| | |
|---|---|
| Valepotriatgemisch | 240 Gew.-Teile |
| Miglyol 812 | 222 Gew.-Teile |
| Gesamt | 462 Gew.-Teile |

Das Valepotriatgemisch wird unter leichtem Erwärmen und Rühren in dem Miglyol 812 aufgelöst und die Lösung wird zu im Mittel 462 Mikroliter Lösung enthaltenden Weichgelatinekapseln verarbeitet. Valepotriatwirkstoffgehalt pro Kapsel 240 mg.

C) Kapseln mit einem Valepotriat-Gehalt von 320 mg

Zusammensetzung:

| | |
|---|---|
| Valepotriatgemisch | 320 Gew.-Teile |
| Miglyol 812 | 296 Gew.-Teile |
| Gesamt | 616 Gew.-Teile |

Das Valepotriatgemisch wird unter leichtem Erwärmen und Rühren in dem Miglyol 812 gelöst und die Lösung wird zu im Mittel 616 Mikroliter Lösung enthaltenden Weichgelatinekapseln verarbeitet. Valepotriatwirkstoffgehalt pro Kapsel 320 mg.

D) Kapseln mit einem Valepotriatgehalt von 500 mg

Zusammensetzung:

| | |
|---|---|
| Valepotriatgemisch | 500 Gew.-Teile |
| Miglyol 812 | 424 Gew.-Teile |
| Gesamt | 924 Gew.-Teile |

Das Valepotriatgemisch wird unter leichtem Erwärmen und Rühren in dem Miglyol 812 aufgelöst. Die Lösung wird zu im Mittel 924 Mikroliter Lösung enthaltenden Weichgelatinekapseln verarbeitet. Valepotriatwirkstoffgehalt pro Kapsel 500 mg.

KC8306EP01

## Patentansprüche

1. Verwendung von Valepotriaten zur Behandlung von Tumoren in Menschen und größeren Säugetieren, dadurch gekennzeichnet, daß man ein oder mehrere Valepotriate in tumorhemmend wirksamen Tagesdosen von mindestens 700 mg pro Tag appliziert.

2. Verwendung von Valepotriaten gemäß Anspruch 1, dadurch gekennzeichnet, daß die Valepotriate in Tagesdosen von 700-2100 mg, vorzugsweise 800-1500 mg, angewendet werden.

3. Verwendung von Valepotriaten gemäß Anspruch 1, dadurch gekennzeichnet, daß die Valepotriate oral appliziert werden.

4. Verwendung von Valepotriaten gemäß Anspruch 1, dadurch gekennzeichnet, daß Valepotriate mit Monoenstruktur, insbesondere Didrovaltratum, gegebenenfalls im Gemisch mit anderen Valepotriaten, verwendet werden.

5. Verwendung von Valepotriaten gemäß Anspruch 1, dadurch gekennzeichnet, daß ein Valepotriatgemisch verwendet wird, welches mindestens 50 % monoenische Valepotriate, insbesondere Didrovaltratum, enthält.

6. Verwendung von Valepotriaten gemäß Anspruch 5, dadurch gekennzeichnet, daß ein Valepotriatgemisch verwendet wird, welches 70-90 % Didrovaltratum, 10-30 % dienische Valepotriate, insbesondere Isovaltratum und/oder Valtratum, enthalten.

7. Verwendung von Valepotriaten gemäß Anspruch 5, dadurch gekennzeichnet, daß ein Valepotriatgemisch verwendet wird, welches Didrovaltratum und dienische Valepotriate in einem der natürlichen Valepotriatzusammensetzungen in Valeriana wallichii D.C. entsprechendem Mengenverhältnis enthält.

8. Verwendung von Valepotriaten gemäß Anspruch 1, dadurch gekennzeichnet, daß die Valepotriate zur prophylaktischen und kurativen Behandlung von malignen Tumoren, insbesondere Karzinomen, verwendet werden.

9. Verwendung von Valepotriaten gemäß Anspruch 8 zur prophylaktischen und kurativen Behandlung von gegebenenfalls metastasierenden Leberkarzinomen, Mammakarzinomen oder Lungenkarzinomen.

10. Verwendung von Valepotriaten gemäß Anspruch 1 oder 2, dadurch gekennzeichnet, daß zusätzlich zu den Valepotriaten Natriumchlorid in Tagesdosen von 1,5-4 g appliziert wird.

11. Verwendung von Valepotriaten gemäß Anspruch 10, dadurch gekennzeichnet, daß das Verhältnis von Valepotriaten zu Natriumchlorid von 1 : 1 bis 1 : 5 beträgt.

12. Arzneimittel mit tumorhemmender Wirkung, dadurch gekennzeichnet, daß sie neben üblichen pharmazeutischen Wirkstoffen als tumorhemmenden Wirkstoff ein oder mehrere Valepotriate in solchen Mengen enthalten, welche eine turmorwirksame Tagesdosis von mindestens 700 mg pro Tag ergeben.

13. Einzeldosierte Arzneimittel gemäß Anspruch 12, dadurch gekennzeichnet, daß sie eine Menge von 100-800 mg Valepotriate pro Dosiseinheit enthalten.

14. Arzneimittel gemäß Anspruch 13, dadurch gekennzeichnet, daß mindestens ein Teil der darin enthaltenen Valepotriate Valepotriate mit Monoenstruktur sind.

15. Arzneimittel gemäß Anspruch 14, dadurch gekennzeichnet, daß sie ein Valepotriatgemisch enthalten, welches mindestens 50 % monoenische Valepotriate, insbesondere Didrovaltratum, enthält.

16. Arzneimittel gemäß Anspruch 15, dadurch gekennzeichnet, daß sie ein Valepotriatgemisch enthalten, welches 70-90 % Didrovaltratum, 10-25 % Isovaltratum und/ oder Valtratum und 1-7 % Acevaltratum enthält.

17. Arzneimittel gemäß Anspruch 16, dadurch gekennzeichnet, daß sie ein Valepotriatgemisch enthalten, welches Didrovaltratum und dienische Valepotriate in einem der natürlichen Valepoptriatzusammensetzungen in Valeriana wallichii D.C. entsprechenden Merngenverhältnis enthält.

18. Oral applizierbare Arzneimittel gemäß Anspruch 12, dadurch gekennzeichnet, daß sie einen magensaftresistenten Überzug haben.

19. Arzneimittel gemäß Anspruch 12 zur prophylaktischen und kurativen Behandlung von malignen Tumoren, insbesondere Karzinomen.

20. Arzneimittel gemäß Anspruch 19 zur prophylaktischen und kurativen Behandlung von gegebenenfalls metastasierenden Leberkarzinomen, Mammakarzinomen oder Lungenkarzinomen.

21. Behältnis enthaltend Arzneimittel gemäß Anspruch 12 und eine Anweisung zur Einnahme dieser Arzneimittel zur prophylaktischen und kurativen Behandlungen von Tumoren.